# EUROPÄISCHE PATENTSCHRIFT

(11) **EP 3 189 122 B1**
(45) Veröffentlichungstag und Bekanntmachung des Hinweises auf die Patenterteilung: **13.06.2018**
(21) Anmeldenummer: 15754128.5
(22) Anmeldetag: 31.07.2015
(51) Int. Cl.: C10L 3/00, C07C 29/00

(54) **INDUSTRIELLE PRODUKTIONSANLAGE MIT MINIMALEM TREIBHAUSGASAUSSTOSS, INSBESONDERE KOHLENDIOXIDAUSSTOSS, UND VERFAHREN ZUM BETRIEB DERSELBEN**
INDUSTRIAL PRODUCTION PLANT HAVING MINIMAL EMISSION OF GREENHOUSE GASES, IN PARTICULAR EMISSION OF CARBON DIOXIDE, AND METHOD FOR THE OPERATION THEREOF
UNITE DE PRODUCTION INDUSTRIELLE À REJET DE GAZ A EFFET DE SERRE, EN PARTICULIER A REJET DE DIOXYDE DE CARBONE, MINIMAL ET PROCEDE POUR FAIRE FONCTIONNER CELLE-CI

(30) Priorität: 01.09.2014 DE 102014112580
(43) Veröffentlichungstag der Anmeldung: 12.07.2017
(73) Patentinhaber: Mitsubishi Hitachi Power Systems Europe GmbH, 47059 Duisburg (DE)
(72) Erfinder: BERGINS, Christian, 45711 Datteln (DE); BUDDENBERG, Torsten, 47447 Moers (DE); KOYTSOUMPA, Efthymia-Ioanna, 47051 Duisburg (DE)
(74) Vertreter: Viering, Jentschura & Partner mbB Patent- und Rechtsanwälte
(86) Internationale Anmeldenummer: PCT/EP2015/067708
(87) Internationale Veröffentlichungsnummer: WO 2016/034344

(56) Entgegenhaltungen:
- WO-A1-2013/029701
- DE-A1-102009 018 126
- FR-A1- 2 977 089
- US-A1- 2011 041 740
- US-A1- 2011 237 839

## Beschreibung

Die Erfindung richtet sich auf eine industrielle Produktionsanlage, die eine aus einem kohlenstoffhaltigen Einsatzstoff ein CO₂-armes und H₂-reiches Produktgas oder Abgas erzeugende erste Produktionsanlage, insbesondere einen Koksofen, mit einer zugeordneten ersten Abgasreinigungsvorrichtung und einer zugeordneten zweiten Abgasreinigungsvorrichtung umfasst.

Weiterhin richtet sich die Erfindung auf ein Verfahren zum zumindest im Wesentlichen CO₂-freien Betrieb einer aus einem kohlenstoffhaltigen Einsatzstoff ein CO₂-armes und H₂-reiches Produktgas oder Abgas erzeugenden ersten Produktionsanlage mit einer zugeordneten ersten Abgasreinigungsvorrichtung und einer zugeordneten zweiten Abgasreinigungsvorrichtung.

Es ist ein seit längerem bestehendes allgemeines Bestreben, industrielle Produktionsanlagen mit einem möglichst geringen Treibgasausstoß, insbesondere einem möglichst geringen CO₂-Ausstoß zu betreiben. Es sind daher bereits eine Reihe von Verfahren und Vorrichtungen entwickelt worden, mit Hilfe welcher das jeweilige Treibhausgas und insbesondere Kohlendioxid aus dem Abgas herausgefiltert und gespeichert werden kann. Diese häufig auch unter dem Oberbegriff "Carbon Capture and Storage" subsummierten Technologien, Verfahren und Vorrichtungen zielen darauf ab, Kohlendioxid von einem Abgas, insbesondere einem Rauchgas, abzuscheiden und dann zu lagern. Da dieser Abscheidungsprozesse jeweils Wirkungsgradverluste oder einen erhöhten Investitionsaufwand und damit verbundene zusätzliche Kosten verursachen, sind im Stand der Technik auch schon Konzepte veröffentlicht worden, die unter dem Begriff "Carbon Capture and Utilisation" subsummiert werden und welche nicht die reine Lagerung des Kohlendioxids, sondern dessen Weiterverarbeitung zu einem handelbaren Produkt offenbaren. Ein solches Konzept stellt beispielsweise das so genannte "Power to Fuel"-Konzept dar, bei welchem der CO₂-Anteil in Rauchgasen von Kraftwerken unter Hinzuziehung von in einer zugeordneten Wasserelektrolyse erzeugtem Wasserstoff (H₂) zu Methan, Methanol oder Methan- oder Methanolfolgeprodukten umgewandelt wird.

Aus der WO 2013/029701 A1 ist eine hocheffiziente, dezentrale und weitgehend CO₂ neutrale sowie vorzugsweise autarke Energieversorgungsanlage insbesondere für den Bereich der Haustechnik bekannt. Die Anlage umfasst einen Reformer, der aus einem kohlenstoffhaltigen Einsatzstoff, z.B. Erdgas, ein Kohlendioxid und Wasserstoff umfassendes Gas erzeugt. Bei dem Reformer handelt es sich um einen Dampfreformer, alternativ kann eine Brennstoffzelle vorgesehen sein. Weiter umfasst die Anlage einen Gasseparator, der das erzeugte Gas in einen CO₂-haltigen, H₂-freien Teilgasstrom und einen CO₂-freien, H₂-reichen Teilgasstrom aufteilt. Ferner umfasst die Anlage eine Wasserstoff und Sauerstoff erzeugende Elektrolyseanlage und eine Methanol erzeugende Produktionsanlage. Die Produktionsanlage steht in CO₂-Leitungsverbindung mit einem CO₂-Tank, wobei der Produktionsanlage mittels der CO₂-Leitungsverbindung aus dem CO₂-Tank der CO₂-reiche Gasstrom zuführbar ist. Die Produktionsanlage steht andererseits in H₂-Leitungsverbindung mit der Gasaufbereitungsanlage und der Elektrolyseanalage und ihr ist mittels dieser H₂-Leitungsverbindung der in der Gasaufbereitungsanlage erzeugte H₂-reiche Teilgasstrom und/oder der in der Elektrolyseanlage erzeugte Wasserstoff zumindest jeweils teilweise zuführbar.

Eine industrielle Produktionsanlage zur Herstellung von Methanol mit einem rekuperativen Verbrennungssystem, das Energie aus der Verbrennung, die sonst verloren gehen würde, rekuperiert, offenbart weiterhin die US 2011/0041740 A1. Die industrielle Produktionsanlage umfasst eine Produktionsanlage, die aus einem kohlenstoffhaltigem Einsatzstoff ein CO₂-reiches Produkt oder Abgas erzeugt. Ferner umfasst die Produktionsanlage eine Feuerungseinrichtung, der eine Abgasreinigungsvorrichtung zugeordnet ist. Weiterhin umfasst die Produktionsanlage eine Wasserelektrolyseanlage zur Erzeugung von Wasserstoff und Sauerstoff sowie einen Methanolreaktor. Dieser steht in H₂-Leitungsverbindung mit der Wasserelektrolyseanlage, sodass der durch die Wasserelektrolyseanlage erzeugte Wasserstoff dem Methanolreaktor zuführbar ist.

Vor diesem Hintergrund liegt der Erfindung die Aufgabe zugrunde, eine Lösung zu schaffen, die eine vorteilhaft realisierbare und durchführbare Alternative für eine industrielle Produktionsanlage bereitstellt, mit welcher ein Carbon Capture and Utilisation-Verfahren effektiv und effizient durchführbar ist.

Die vorstehende Aufgabe wird gelöst durch eine industrielle Produktionsanlage gemäß Anspruch 1 und ein Verfahren gemäß Anspruch 10.

Demnach umfasst die erfindungsgemäße industrielle Produktionsanlage eine aus einem kohlenstoffhaltigen Einsatzstoff ein CO₂-armes und H₂-reiches Produktgas oder Abgas erzeugende erste Produktionsanlage mit einer zugeordneten ersten Abgasreinigungsvorrichtung und einer zugeordneten zweiten Abgasreinigungsvorrichtung,
eine über eine erste Leitungsverbindung mit der ersten Abgasreinigungsvorrichtung verbundene, das Produkt- oder Abgas in einen kohlenstoffhaltigen, zumindest im Wesentlichen H₂-freien und einen zumindest im Wesentlichen kohlenstofffreien, H₂-reichen Teilgasstrom aufteilende Gasaufbereitungsanlage, wobei der im Wesentlichen H₂-freie Teilgasstrom mittels einer zweiten Leitungsverbindung zumindest teilweise einer oder mehreren Feuerungseinrichtung(en) zuführbar ist, welche die erste Produktionsanlage aufweist,
eine Einrichtung zur Erzeugung eines CO₂-reichen Gasstroms, der zumindest ein Teil des in der oder den Feuerungseinrichtung(en) entstehenden CO₂-haltigen Abgasstroms nach Durchströmen der zweiten Abgasreinigungsvorrichtung zumindest teilweise zuführbar ist,
eine Wasserstoff (H₂) und Sauerstoff (O₂) erzeugende Wasserelektrolyseanlage und eine Methanol und/oder Methanolfolgeprodukte erzeugende zweite Produktionsanlage, die einerseits in CO₂-Leitungsverbindung mit der den CO₂-reichen Gasstroms erzeugenden Einrichtung steht und der mittels dieser CO₂-Leitungsverbindung der CO₂-reiche Gasstrom zumindest teilweise zuführbar ist und die andererseits in H₂-Leitungsverbindung mit der Gasaufbereitungsanlage und der Wasserelektrolyseanlage steht und der mittels dieser H₂-Leitungsverbindung der in der Gasaufbereitungsanlage erzeugte H₂-reiche Teilgaststrom und/oder der in der Wasserelektrolyseanlage erzeugte Wasserstoff (H₂) zumindest jeweils teilweise zuführbar ist.

Das erfindungsgemäße Verfahren zum zumindest im Wesentlichen CO₂-freien Betrieb einer aus einem kohlenstoffhaltigen Einsatzstoff ein CO₂-armes und H₂-reiches Produktgas oder Abgas erzeugenden ersten Produktionsanlage mit einer zugeordneten ersten Abgasreinigungsvorrichtung und einer zugeordneten zweiten Abgasreinigungsvorrichtung, insbesondere zum Betrieb einer industriellen Produktionsanlage nach einem der Ansprüche 1 - 9, zeichnet sich dadurch aus, dass das CO₂-arme und H₂-reiche Produktgas oder Abgas über eine erste, mit der ersten Abgasreinigungsvorrichtung verbundene Leitungsverbindung einer das Produktgas oder Abgas in einen kohlenstoffhaltigen, zumindest im Wesentlichen H₂-freien und einen zumindest im Wesentlichen kohlenstofffreien, H₂-reichen Teilgasstrom aufteilenden Gasaufbereitungsanlage zugeführt wird und der im Wesentlichen H₂-freie Teilgasstrom über eine zweite Leitungsverbindung einer oder mehreren Feuerungseinrichtung(en) der ersten Produktionsanlage zumindest teilweise zugeleitet wird,
wobei zumindest ein Teil des in der oder den Feuerungseinrichtung(en) entstehenden CO₂-haltigen Abgasstroms nach Durchlaufen der zweiten Abgasreinigungsvorrichtung zumindest teilweise einer Einrichtung zur Erzeugung eines CO₂-reichen Gasstroms zugeführt wird,
wobei in einer Wasserelektrolyseanlage mittels Wasserelektrolyse Wasserstoff (H₂) sowie Sauerstoff (O₂) und in einer zweiten Produktionsanlage Methanol und/oder Methanolfolgeprodukte erzeugt werden und der zweiten Produktionsanlage einerseits über eine CO₂-Leitungsverbindung der in der den CO₂-reichen Gasstrom erzeugenden Einrichtung erzeugte CO₂-reiche Gasstrom zumindest teilweise zugeführt wird und der andererseits über eine H₂-Leitungsverbindung der von der Gasaufbereitungsanlage erzeugte H₂-reiche Teilgaststrom und/oder der in der Wasserelektrolyseanlage erzeugte Wasserstoff (H₂) zumindest jeweils teilweise zugeführt wird.

Die erfindungsgemäße industrielle Produktionsanlage und das erfindungsgemäße Verfahren bieten den Vorteil, dass damit 90 % des in einer aus einem kohlenstoffhaltigen Einsatzstoff ein CO₂-armes und H₂-reiches Produktgas oder Abgas erzeugenden ersten Produktionsanlage erzeugte Kohlendioxid, aber auch das in der industriellen Produktionsanlage insgesamt entstehende CO₂, weder in die Umwelt entlassen noch gespeichert, sondern unmittelbar zu einem handelbaren und verkaufsfähigen Produkt, nämlich Methanol oder ein Methanolfolgeprodukt, umgewandelt werden. Durch die unmittelbare Erzeugung des handelbaren Produktes Methanol oder ein Methanolfolgeprodukt wird die Wirtschaftlichkeit einer solchen industriellen Produktionsanlage verbessert. Hierzu trägt u. a. auch bei, dass der derzeit noch häufig im öffentlichen Stromnetz vorhandene so genannte "Überschussstrom", der aufgrund einer Überproduktion der auf Basis erneuerbarer Energien stromproduzierenden Erzeugungsanlagen häufig vorhanden ist, für die vorgesehene Wasserelektrolyse genutzt werden kann. Somit kann dann ein konventionelles Kraftwerk, insbesondere ein Kohlekraftwerk, im Rahmen dieser industriellen Produktionsanlage ebenfalls wirtschaftlich auch in solchen Fällen betrieben werden, da der im Mindestlastbereich weiterhin entstehende Strom in der Wasserelektrolyse genutzt werden kann, die zeitlich sehr flexibel hoch- und heruntergefahren werden kann. Insgesamt kann sich bei entsprechender Nutzung und Rückintegration entstehender Abwärmen in den im Rahmen der erfindungsgemäßen industriellen Produktionsanlage möglichen Produktionsprozess ein Wirkungsgrad von 69 % der Produktionsanlage erzielen.

In Ausgestaltung der industriellen Produktionsanlage sieht die Erfindung daher auch vor, dass sie ein mit kohlenstoffhaltigem Brennstoff befeuertes Kraftwerk umfasst, das einen Wasser/Dampfkreislauf aufweist, der mindestens einen dampfbeaufschlagten Turbosatz mit mindestens einem Generator umfasst, und das über eine dritte Leitungsverbindung mit der Gasaufbereitungsanlage in medienführender Leitungsverbindung steht, mittels welcher der im Wesentlichen H₂-freie Teilgasstrom zumindest teilweise dem Kraftwerk zuführbar ist.

In analoger Weise zeichnet sich das erfindungsgemäße Verfahren in Weiterbildung dadurch aus, dass der im Wesentlichen H₂-freie Teilgasstrom über eine dritte Leitungsverbindung zumindest teilweise einem Kraftwerk zugeleitet wird, wobei das Kraftwerk einen Wasser/Dampfkreislauf mit mindestens einem dampfbeaufschlagten Turbosatz mit mindestens einem Generator aufweist und mit kohlenstoffhaltigem Brennstoff befeuert wird sowie sein CO₂-haltiger Abgasstrom zumindest teilweise der zugeordneten zweiten Abgasreinigungsvorrichtung zugeführt wird.

Um die Flexibilität des Kraftwerkes und die flexible Nutzung von im öffentlichen Stromnetz gegebenenfalls vorhandenem Überschussstrom nutzen zu können, sieht die Erfindung in vorteilhafter Weiterbildung vor, dass die Gasaufbereitungsanlage und die Wasserelektrolyseanlage eine elektrische Leitungsverbindung sowohl zu dem mindestens einen Generator des Kraftwerks als auch zum öffentlichen Stromnetz aufweisen und wahlweise mit im öffentlichen Stromnetz vorhandenem Strom, insbesondere Überschussstrom, und/oder mit von dem mindestens einen Generator erzeugtem elektrischem Strom betreibbar sind.

Aufgrund dieser Vernetzung kann die einerseits vom Kraftwerk erzeugte Strommenge und andererseits die aus dem Netz beispielsweise im Rahmen der Regelungsleistung eines Kraftwerkes abgenommene Leistung flexibel auf die Gasaufbereitungsanlage und die Wasserelektrolyse verteilt und gegebenenfalls die Wasserelektrolyse kurzfristig hoch oder herunter gefahren werden.

Insbesondere dann, wenn die aus einem kohlenstoffhaltigen Einsatzstoff ein CO₂-armes und H₂-reiches Produktgas oder Abgas erzeugende erste Produktanlage Feuerungseinrichtungen aufweist, wie dies beispielsweise bei einem Koksofen der Fall ist, lässt sich der in der Gasaufbereitungsanlage erzeugte, zumindest im Wesentlichen H₂-freie und in der Regel kohlenstoffhaltige Teilgasstrom vorteilhaft in einer solchen Feuerungsanlage verbrennen. Dies gilt insbesondere dann, wenn dieser Teilgasstrom auch noch Methananteile enthält, wie dies bei einem Kokereigas der Fall ist. Die Erfindung zeichnet sich daher weiterhin dadurch aus, dass die eine oder mehrere Feuerungseinrichtungen der ersten Produktionsanlage einlassseitig über die zweite Leitungsverbindung und/oder die dritte Leitungsverbindung in medienführender Leitungsverbindung mit der Gasaufbereitungsanlage steht/stehen, über welche der oder den Feuerungseinrichtung(en) zumindest ein Teil des in der Gasaufbereitungsanlage erzeugten kohlenstoffhaltigen, zumindest im Wesentlichen H₂-freien Teilgasstroms als Brennstoff zuführbar ist.

Zweckmäßig und besonders vorteilhaft ist es dann weiterhin ebenso, wenn das insbesondere in solchen Feuerungseinrichtungen, wie beispielsweise der oder den Feuerungseinrichtung(en) eines Koksofens entstehende, ebenfalls CO₂-haltige Abgas aufbereitet und genutzt wird. Die Erfindung sieht in weiterer Ausgestaltung daher ebenfalls vor, dass die eine oder mehrere Feuerungseinrichtungen der ersten Produktionsanlage abgasseitig über eine vierte Leitungsverbindung mit der zweiten Abgasreinigungsvorrichtung und/oder mit einer den CO₂-haltigen Abgasstrom des Kraftwerks führenden Leitungsverbindung in medienführender Leitungsverbindung steht/stehen, über welche der zweiten Abgasreinigungsvorrichtung zumindest ein Teil der in der oder den Feuerungseinrichtung(en) und/oder dem Kraftwerk entstehenden CO₂-haltigen Abgasströme, vorzugsweise die Abgasströme insgesamt, zuführbar ist.

Eine besonders zweckmäßige Ausführung einer Gasaufbereitungsanlage stellt eine Druckwechseladsorptionsanlage dar, so dass die Erfindung in weiterer Ausgestaltung zudem vorsieht, dass die Gasaufbereitungsanlage als Druckwechseladsorptionsanlage ausgebildet ist.

Um aus dem sich bildenden Produktgas/Abgas/Rauchgas-Strom das Kohlendioxid herauslösen und von diesem abtrennen zu können, haben sich so genannte Post Combustion Capture (PCC)-Verfahren als besonders vorteilhaft erwiesen. Die Erfindung zeichnet sich in weiterer Ausgestaltung daher auch dadurch aus, dass die einen CO₂-reichen Gasstrom erzeugende Einrichtung als CO₂ mittels eines Absorptionsmittels aus dem CO₂-haltigen Abgasstrom des Kraftwerks und/oder der einen oder mehreren Feuerungseinrichtung(en) waschende Post Combustion Capture (PCC)-Anlage ausgebildet ist.

Das erfindungsgemäße Verfahren kann bei einer Vielzahl von industriellen Produktionsanlagen vorteilhaft angewendet werden. Insbesondere solche industriellen Produktionsanlagen sind vorteilhaft, bei welchen die aus einem kohlenstoffhaltigen Einsatzstoff ein CO₂-armes und H₂-reiches Produktgas oder Abgas erzeugende erste Produktionsanlage ein Kokereigas erzeugender Koksofen oder eine kohlenstoffhaltiges Ausgangsmaterial vergasende oder pyrolysierende oder torrefizierende Anlage ist, was die Erfindung ebenfalls vorsieht.

Bei der industriellen Produktionsanlage kann es sich um eine Vielzahl an Produktionsanlagen handeln. In Ausgestaltung der Erfindung ist insbesondere vorgesehen, dass die industrielle Produktionsanlage ein Hüttenwerk und/oder ein Stahlwerk oder ein Zementwerk, insbesondere einen Drehrohrofen, oder eine oder mehrere Anlagen zur Herstellung von schmelzflüssigem Glas oder ein Chemiewerk oder eine oder mehrere Papierherstellungsanlagen umfasst oder mit diesen/diesem/dieser in einer einen CO₂-haltigen Gasstrom führenden Leitungsverbindung steht.

In zweckmäßiger Ausgestaltung des Verfahrens sieht die Erfindung vor, dass die Gasaufbereitungsanlage und die Wasserelektrolyseanlage in elektrischer Leitungsverbindung sowohl zu dem mindestens einen Generator des Kraftwerks als auch zum öffentlichen Stromnetz stehen und sie wahlweise mit im öffentlichen Stromnetz vorhandenem Strom, insbesondere Überschussstrom, und/oder mit von dem mindestens einen Generator erzeugtem elektrischem Strom betrieben werden. Hierdurch wird derselbe Vorteil, wie vorstehend zur industriellen Produktionsanlage ausgeführt, erzielt, nämlich die flexible Nutzung von erzeugtem oder aus dem öffentlichen Netz zur Verfügung stehendem elektrischem Strom und eine damit verbundene Flexibilisierung des Kraftwerkbetriebes.

Von Vorteil ist es auch bei dem Verfahren, wenn die ein CO₂-armes und H₂-reiches Produktgas oder Abgas erzeugende erste Produktionsanlage eine oder mehrere Feuerungseinrichtungen aufweist, der/denen einlassseitig von der Gasaufbereitungsanlage und/oder der zweiten Leitungsverbindung zumindest ein Teil des in der Gasaufbereitungsanlage erzeugten kohlenstoffhaltigen, zumindest im Wesentlichen H₂-freien Teilgasstroms als Brennstoff zugeführt wird.

Auch hier ergeben sich die vorstehend zur industriellen Produktionsanlage aufgeführten Vorteile in gleicher Weise.

Dies gilt schließlich auch für die weitere Ausgestaltung der Erfindung, wonach die ein CO₂-armes und H₂-reiches Produktgas oder Abgas erzeugende erste Produktionsanlage eine oder mehrere Feuerungseinrichtungen aufweist, die abgasseitig über eine vierte Leitungsverbindung mit der zweiten Abgasreinigungsvorrichtung und/oder mit einer den CO₂-haltigen Abgasstrom des Kraftwerks führenden Leitungsverbindung in medienführender Leitungsverbindung steht/stehen, über welche der zweiten Abgasreinigungsvorrichtung zumindest ein Teil der in der oder den Feuerungseinrichtung(en) und/oder dem Kraftwerk entstehenden CO₂-haltigen Abgasströme, vorzugsweise die CO₂-haltigen Abgasströme insgesamt, zugeführt wird.

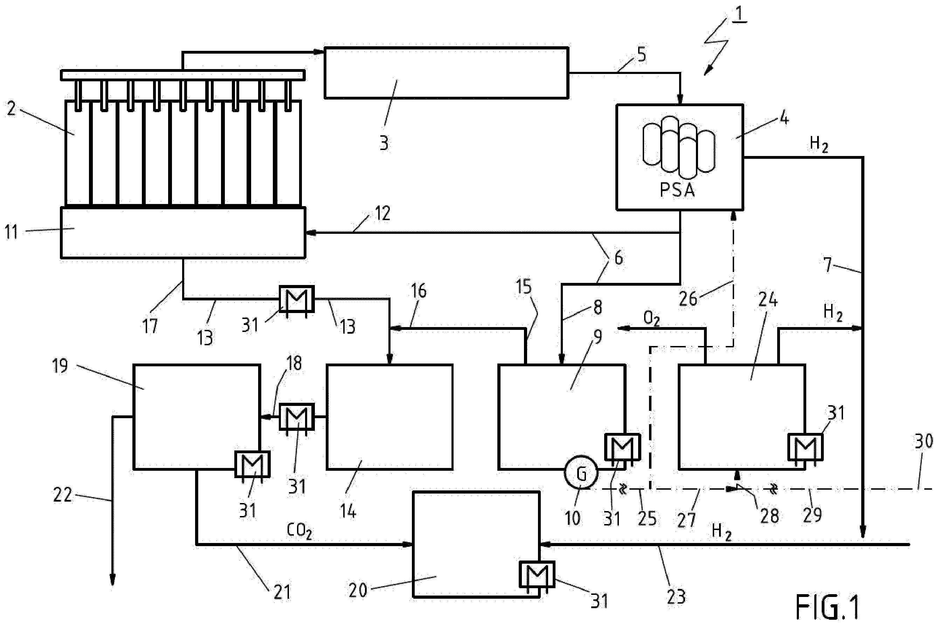

Die Erfindung ist nachstehend anhand einer Zeichnung beispielhaft näher erläutert. Diese zeigt in der einzigen Figur in schematischer Zusammenstellung eine erfindungsgemäße industrielle Produktionsanlage, mit welcher das erfindungsgemäße Verfahren durchführbar ist.

Die in der einzigen Figur insgesamt mit 1 bezeichnete industrielle Produktionsanlage umfasst eine aus einem kohlenstoffhaltigen Einsatzstoff ein CO₂-armes und H₂-reiches Produktgas oder Abgas erzeugende erste Produktionsanlage 2. Im Ausführungsbeispiel handelt es sich bei der ersten Produktionsanlage 2 um einen Koksofen, so dass das hier erzeugte CO₂-arme und H₂-reiche Produktgas oder Abgas ein Kokereigas ist. Ein solches Kokereigas weist zudem Methan als Bestandteil auf. Der ersten Produktionsanlage 2 ist eine erste Abgasreinigungsvorrichtung 3 zugeordnet, welcher das in der ersten Produktionsanlage 2 erzeugte Produktgas oder Abgas zugeführt wird. In dieser ersten Abgasreinigungsvorrichtung 3 wird das Produktgas oder Abgas, hier Kokereigas, insoweit gereinigt, als es in einer nachfolgenden Gasaufbereitungsanlage 4 weiterverarbeitet werden kann. Hierzu wird das Produktgas oder Abgas in der ersten Abgasreinigungsvorrichtung 3 in der Regel entstaubt und ggf. von sauren Bestandteilen befreit. Der Gasaufbereitungsanlage 4 wird das Produktgas oder Abgas über eine erste Leitungsverbindung 5 zugeführt, die einerseits mit der ersten Abgasreinigung 3 und andererseits mit der Gasaufbereitungsanlage 4 in medienführender Weise verbunden ist. In der Gasaufbereitungsanlage 4 wird der zugeführte CO₂-arme und H₂-reiche Produktgas- oder Abgasstrom bzw. das Produkt oder Abgas in einen kohlenstoffhaltigen, zumindest im Wesentlichen H₂-freien Teilgasstrom 6 und einen zumindest im Wesentlichen kohlenstofffreien, H₂-reichen Teilgasstrom 7 aufgeteilt. Bei der Gasaufbereitungsanlage 4 handelt es sich im Ausführungsbeispiel um eine Druckwechseladsorptionsanlage, wie sie hinlänglich aus dem Stand der Technik bekannt ist. Über eine dritte Leitungsverbindung 8 steht die Gasaufbereitungsanlage 4 in medienführender Leitungsverbindung mit einem Kraftwerk 9, so dass diesem der in der Gasaufbereitungsanlage 4 erzeugte, zumindest im Wesentlichen H₂-freie und üblicherweise kohlenstoffhaltige Teilgasstrom 6 zumindest teilweise zugeführt und dort insbesondere den Brennern zur Unterstützung der Verbrennung von Brennstoff als Brennstoffzusatz oder Brennstoffersatz zugeführt wird oder zumindest zugeführt werden kann.

Bei dem Kraftwerk 9 handelt es sich um ein mit kohlenstoffhaltigem Brennstoff, insbesondere Gas oder Kohle, befeuertes Kraftwerk, das in üblicher Weise einen nicht dargestellten Wasser/Dampfkreislauf aufweist, der mindestens einen dampfbeaufschlagten Turbosatz und mindestens einen Generator 10 umfasst. Als kohlenstoffhaltiger Brennstoff kann aber auch Biomasse Verwendung finden.

Die erste Produktionsanlage 2 weist eine Feuerungseinheit oder Feuerungseinrichtung 11 auf, in welcher zumindest ein Teil des in der Gasaufbereitungsanlage 4 erzeugten kohlenstoffhaltigen, zumindest im Wesentlichen H₂-freien Teilgasstromes 6 verbrannt wird oder zur Unterstützung der Verbrennung Verwendung findet. Hierzu steht die Feuerungseinheit oder Feuerungseinrichtung 11 einlassseitig über eine zweite Leitungsverbindung 12 mit der Gasaufbereitungsanlage 4 und/oder der dritten Leitungsverbindung 8 in medienführender Leitungsverbindung. Vorteilhafterweise wird von dem in der Gasaufbereitungsanlage 4 erzeugten kohlenstoffhaltigen, zumindest im Wesentlichen H₂-freien Teilgasstrom 6 eine solche Menge der Feuerungseinrichtung 11 zugeführt, wie dort zur Erzeugung der benötigten Wärme und Feuerungsleistung benötigt wird. Lediglich der verbleibende Restanteil an in der Gasaufbereitungsanlage 4 erzeugtem kohlenstoffhaltigem, zumindest im Wesentlichen H₂-freiem Teilgasstrom 6 wird dann via dritter Leitungsverbindung 8 dem Kraftwerk 9 zugeführt.

Abgasseitig steht die Feuerungseinrichtung 11 der ersten Produktionsanlage 2 über eine den CO₂-haltigen Abgasstrom 17 Feuerungseinrichtung 11 führende vierte Leitungsverbindung 13 in medienführender Leitungsverbindung mit einer zweiten Abgasreinigungsvorrichtung 14, welcher das in der Feuerungseinrichtung 11 entstehende Abgas zugeführt wird. Ebenso steht die zweite Abgasreinigungsvorrichtung 14 über eine den CO₂-haltigen Abgasstrom 15 des Kraftwerks 9 führende Leitungsverbindung 16 in medienführender Leitungsverbindung mit dem Kraftwerk 9. In der zweiten Abgasreinigungsvorrichtung 14 werden die CO₂-haltigen Abgasströme 15 und 17 von Staubpartikeln und weitestgehend von sauren Bestandteilen wie SOₓ und NOₓ befreit.

Über eine fünfte Leitungsverbindung 18 wird der derart gereinigte CO₂-haltige Abgasstrom einer Einrichtung 19 zur Erzeugung eines CO₂-reichen Gasstroms zugeführt. Bei dieser Einrichtung 19 handelt es sich um eine so genannte Post Combustion Capture (PCC) Anlage, in welcher das im Abgasstrom enthaltene CO₂ mittels eines Absorptionsmittels aus dem CO₂-haltigen Abgasstrom 15 des Kraftwerks 9 und dem CO₂-haltigen Abgasstrom 17 der Feuerungseinrichtung 11 ausgewaschen wird, wenn diese die zweite Abgasreinigungsvorrichtung 14 durchströmen.

Die industrielle Produktionsanlage 1 umfasst ferner eine Methanol- und/oder Methanolfolgeprodukte erzeugende zweite Produktionsanlage 20, welcher über eine CO₂-Leitungsverbindung 21 der in der den CO₂-reichen Gasstrom erzeugenden Einrichtung 19 erzeugte CO₂-reiche Gasstrom zugeführt wird. Der weitere, von dem CO₂-befreite Gasstrom verlässt die Einrichtung 19 als Abgas 22.

Die Methanol- und/oder Methanolfolgeprodukte erzeugende zweite Produktionsanlage 20 steht weiterhin über eine H₂-Leitungsverbindung 23 einerseits mit der Wasserstoff abgebenden Seite einer Wasserstoff und Sauerstoff produzierenden Wasserelektrolyseanlage 24 und andererseits mit der den H₂-reichen Teilgasstrom 7 abgebenden Seite der Gasaufbereitungsanlage 4 in medienführender Leitungsverbindung, so dass dieser zweiten Produktionsanlage 20 der in der Wasserelektrolyseanlage 24 erzeugte Wasserstoff (H₂) und/oder der in der Gasaufbereitungsanlage 4 erzeugte H₂-reiche Teilgasstrom 7 zumindest teilweise zuführbar ist. Aus den der Methanol und/oder Methanolfolgeprodukte erzeugenden zweiten Produktionsanlage 20 als Edukte zugeführten Kohlendioxid (CO₂) und Wasserstoff (H₂) wird als Produkt Methanol oder mindestens ein Methanolfolgeprodukt in der zweiten Produktionsanlage 20 hergestellt. Über eine elektrische Leitungsverbindung 25, 26, 27, 28, 29 sind der Generator 10 des Kraftwerks 9, die Gasaufbereitungsanlage 4, die Wasserelektrolyseanlage 24 und das öffentliche Stromnetz 30 elektrisch schalt- und steuerbar miteinander verbunden. Dies ermöglicht es, die jeweils zur Verfügung stehende Strommenge, beispielsweise im öffentlichen Netz vorhandenen Überschussstrom, flexibel für das Hoch- oder Herunterfahren der Gasaufbereitungsanlage 4 und/oder insbesondere der Wasserelektrolyseanlage 24 zu nutzen. Ebenso kann das Kraftwerk 9 auf einer solch hohen Leistungsstufe gehalten werden, dass ein wirtschaftlicher Betrieb möglich ist, da eben immer als Begleitprodukt Methanol oder ein Methanolfolgeprodukt hergestellt wird und die eingesetzte Kraftwerksleistung nicht allein genutzt wird, um elektrischen Strom für das öffentliche Netz oder die sonst bisher im Rahmen einer jeweiligen industriellen Produktionsanlage vorhandenen Vorrichtungen, Einrichtungen oder Anlagen zu produzieren.

In der Einrichtung 19 zur Erzeugung eines CO₂-reichen Gasstroms kann bis zu 90 % des über die fünfte Leitungsverbindung 18 zugeführten Kohlendioxids aus dem zugeführten Gasstrom abgetrennt und der CO₂-Leitungsverbindung 21 zugeführt werden.
Neben dem in der Wasserelektrolyseanlage 24 produzierten Wasserstoff (H₂) kann auch der dort produzierte Sauerstoff (O₂) einer weiteren Verwendung zugeführt werden. Er kann als Oxidationsmittel bei der Verbrennung von Brennstoff im Kraftwerk 9 oder in anderen Anlagen der industriellen Produktionsanlage 1 Verwendung finden.

Der in der als Druckwechseladsorptionsanlage ausgebildeten Gasaufbereitungsanlage 4 als im Wesentlichen kohlenstofffreier, H₂-reicher Teilgasstrom 7 gebildete Wasserstoff (H₂) und ebenso der in der Wasserelektrolyseanlage 24 gebildete Wasserstoff (H₂) werden in üblicher Art und Weise konditioniert und mit dem aus der Einrichtung 19 zur Erzeugung eines CO₂-reichen Gasstroms stammenden druckbeaufschlagten Kohlendioxid (CO₂) gemischt und in der zweiten Produktionsanlage 20 stöchiometrisch mit dem zugeführten Kohlendioxid (CO₂) zu Methanol und/oder Methanolfolgeprodukten umgesetzt.

Bei der Ausbildung der Einrichtung 19 als CO₂ mittels eines Absorptionsmittels aus dem CO₂-haltigen Abgasstrom 15 des Kraftwerks 9 und/oder des CO₂-haltigen Abgasstroms 17 der einen oder mehreren Feuerungseinrichtung(en) 11 waschenden PCC-Anlage wird vorteilhafter Weise ein Amin oder eine Aminlösung als Waschmittel oder Absorptionsmittel verwendet.

Um eine gute Ausnutzung entstehender Produktions- oder Abwärmen zu ermöglichen und Wärmeverschiebungen und/oder Wärmerückintegrationen in verschiedene Stellen der industriellen Produktionsanlage 1 realisieren zu können, sind in der industriellen Produktionsanlage 1 den verschiedenen Leitungen oder Einrichtungen jeweils zugeordnete Wärmetauscher oder Wärmeverschubsysteme ausgebildet, die einheitlich mit dem Bezugszeichen 31 versehen sind.

Bei einem Ausführungsbeispiel eines Koksofens für eine aus einem kohlenstoffhaltigen Einsatzstoff ein CO₂-armes und H₂-reiches Produktgas oder Abgas erzeugende erste Produktionsanlage 2 mit einer Kapazität von ca. 1.630.000 t Koks/Jahr wird ein Teil des erzeugten Koksofengases oder Kokereigases für die zur Befeuerung des Koksofens notwendigen Feuerungseinrichtungen 11 verwendet. Die bei der vorstehenden Gesamtproduktion an Koks entstehende Kokereigasmenge beträgt 394 MWₜₕ oder 9,87 kg/s, wovon 175,62 MWth oder 4,4 kg/s für die Feuerungseinrichtung(en) 11 verwendet werden und der Rest dem Kraftwerk 9 zugeführt wird. Die CO₂ Emission der Feuereinrichtung(en) 11 beträgt dann 6,75 kg/s und die des Kraftwerks 8,14 kg/s.
Nach dem Durchlaufen der ersten Abgasreinigungsvorrichtung 3 weist das Produktgas oder Abgas 23,04 Vol.-% CH₄, 2,69 Vol.-% an anderen Kohlenwasserstoffen, 59,53 Vol.-% H₂, 0,96 Vol.-% CO₂, 3,84 Vol.-% CO, 0,19 Vol.-% O₂, 5,76 Vol.-% N₂ und 3,98 Vol.-% H₂O auf.
In der Gasaufbereitungsanlage 4 werden aus diesem zugeführten CO₂-armen und H₂-reichen Produktgas oder Abgas ca. 94 % des Wasserstoffs (H₂) als H₂ - reicher Teilgasstrom 7 abgetrennt. Der verbleibende im Wesentlichen H₂ - freie Teilgasstrom 6 weist eine Zusammensetzung von 52,33 Vol.-% CH₄, 6,11 Vol.-% an anderen Kohlenwasserstoffen, 8,11 Vol.-% H₂, 2,18 Vol.-% CO₂, 8,72 Vol.-% CO, 0,43 Vol.-% O₂, 13,08 Vol.-% N₂ und 9,04 Vol.-% H₂O auf. Er strömt mit einem Massenfluss von 8,74 kg/s und besitzt einen Heizwert von 29,58 kJ/kg. Dieser im Wesentlichen H₂ - freie Teilgasstrom 6 wird teilweise der Feuerungseinrichtung 11 zugeführt, die einen Massenfluss von 5,94 kg/s oder 175,62 MWₜₕ benötigt und einen Abgas-Massenfluss an CO₂-haltigem Abgasstrom 17 von 10,12 kg/s CO₂ erzeugt, der zusammen mit dem CO₂-haltigen Abgasstrom 15 des Kraftwerks 9 der zweiten Abgasreinigungsvorrichtung 14 zugeführt wird. Dem Kraftwerk 9 werden 82,8 MWₜₕ als verbleibender im Wesentlicher H₂ - freier Teilgasstrom 6 zugeführt, wobei das Kraftwerk 9 einen Massenfluss von 4,77 kg/s CO₂ erzeugt. In der PCC-Anlage 19 werden 90 % des zugeführten und damit 90 % des in der industriellen Produktionsanlage 1 insgesamt produzierten CO₂ mittels einer Aminlösung aus dem Gasstrom ausgewaschen und in einen CO₂ - reichen Gasstrom überführt. Dieser gewonnene CO₂ - reiche Gasstrom wird druckbeaufschlagt und ebenso wie der aus der Gasaufbereitungsanlage 4 als H₂ - reicher Teilgasstrom 7 gewonnene Wasserstoff (H₂) und der in der Wasserelektrolyseanlage 24 gewonnene Wasserstoff (H₂) der Methanol und/oder Methanolfolgeprodukte erzeugenden zweiten Produktionsanlage 20 zugeführt. Aus der Gasaufbereitungsanlage 4 stammt ein H₂ - reicher Teilgasstrom 7 mit einem Massenfluss an 1,13 kg/s oder 135,45 MWₜₕ Wasserstoff (H₂) und aus der Wasserelektrolyseanlage 24 stammt bei einem Einsatz von 62 MWₑ ein Massenfluss an 1,526 kg/s, so dass unter stöchiometrischen Bedingungen in der zweiten Produktionsanlage 20 eine Produktion von 29,15 t Methanol/Std. erfolgt. Bei einer 14 MWₑ - Druckwechseladsorptionsanlage 4 und ohne Abgaswärmenutzung weist dieser erfindungsgemäße Prozess oder dieses Beispiel des erfindungsgemäßen Verfahrens einen Wirkungsgrad von 69 % auf, wohingegen konventionelle Anlagen, die lediglich eine Verbrennung des Kokereigases in dem Kraftwerk 9 vorsehen und keine Methanolproduktion und keine Wasserelektrolyse umfassen, auf einen Wirkungsgrad von lediglich 42 % kommen.

## Patentansprüche

1. Industrielle Produktionsanlage (1) umfassend
eine aus einem kohlenstoffhaltigen Einsatzstoff ein CO₂-armes und H₂-reiches Produktgas oder Abgas erzeugende erste Produktionsanlage (2) mit einer zugeordneten ersten Abgasreinigungsvorrichtung (3) und einer zugeordneten zweiten Abgasreinigungsvorrichtung (14),
eine über eine erste Leitungsverbindung (5) mit der ersten Abgasreinigungsvorrichtung (3) verbundene, das Produkt- oder Abgas in einen kohlenstoffhaltigen, zumindest im Wesentlichen H₂-freien (6) und einen zumindest im Wesentlichen kohlenstofffreien, H₂-reichen (7) Teilgasstrom aufteilende Gasaufbereitungsanlage (4), wobei der im Wesentlichen H₂-freie Teilgasstrom (6) mittels einer zweiten Leitungsverbindung (12) zumindest teilweise einer oder mehreren Feuerungseinrichtung(en) (11) zuführbar ist, welche die erste Produktionsanlage (2) aufweist,
eine Einrichtung (19) zur Erzeugung eines CO₂-reichen Gasstroms, der zumindest ein Teil des in der oder den Feuerungseinrichtung(en) (11) entstehenden CO₂-haltigen Abgasstroms (17) nach Durchströmen der zweiten Abgasreinigungsvorrichtung (14) zumindest teilweise zuführbar ist,
eine Wasserstoff (H₂) und Sauerstoff (O₂) erzeugende Wasserelektrolyseanlage (24)
und eine Methanol und/oder Methanolfolgeprodukte erzeugende zweite Produktionsanlage (20), die einerseits in CO₂-Leitungsverbindung (21) mit der den CO₂-reichen Gasstroms erzeugenden Einrichtung (19) steht und der mittels dieser CO₂-Leitungsverbindung (21) der CO₂-reiche Gasstrom zumindest teilweise zuführbar ist und die andererseits in H₂-Leitungsverbindung (23) mit der Gasaufbereitungsanlage (4) und der Wasserelektrolyseanlage (24) steht und der mittels dieser H₂-Leitungsverbindung (23) der in der Gasaufbereitungsanlage (4) erzeugte H₂-reiche Teilgasstrom (7) und/oder der in der Wasserelektrolyseanlage (24) erzeugte Wasserstoff (H₂) zumindest jeweils teilweise zuführbar ist.

2. Industrielle Produktionsanlage (1) nach Anspruch 1, **dadurch gekennzeichnet, dass** sie ein mit kohlenstoffhaltigem Brennstoff befeuertes Kraftwerk (9) umfasst, das einen Wasser/Dampfkreislauf aufweist, der mindestens einen dampfbeaufschlagten Turbosatz mit mindestens einem Generator (10) umfasst, und das über eine dritte Leitungsverbindung (8) mit der Gasaufbereitungsanlage (4) in medienführender Leitungsverbindung steht, mittels welcher der im Wesentlichen H₂-freie Teilgasstrom (6) zumindest teilweise dem Kraftwerk (9) zuführbar ist.

3. Industrielle Produktionsanlage (1) nach Anspruch 2, **dadurch gekennzeichnet, dass** die Gasaufbereitungsanlage (4) und die Wasserelektolyseanlage (24) eine elektrische Leitungsverbindung (25, 26, 27, 28, 29) sowohl zu dem mindestens einen Generator (10) des Kraftwerks (9) als auch zum öffentlichen Stromnetz (30) aufweisen und wahlweise mit im öffentlichen Stromnetz (30) vorhandenem Strom, insbesondere Überschussstrom, und/oder mit von dem mindestens einen Generator (10) erzeugtem elektrischem Strom betreibbar sind.

4. Industrielle Produktionsanlage (1) nach einem der vorhergehenden Ansprüche, **dadurch gekennzeichnet, dass** die eine oder mehrere Feuerungseinrichtung(en) (11) der erste Produktionsanlage (2) einlassseitig über die zweite Leitungsverbindung (12) und/oder die dritte Leitungsverbindung (8) in medienführender Leitungsverbindung mit der Gasaufbereitungsanlage (4) steht/stehen, über welche der oder den Feuerungseinrichtung(en) (11) zumindest ein Teil des in der Gasaufbereitungsanlage (4) erzeugten kohlenstoffhaltigen, zumindest im Wesentlichen H₂-freien Teilgasstroms (6) als Brennstoff zuführbar ist.

5. Industrielle Produktionsanlage (1) nach einem der vorhergehenden Ansprüche, **dadurch gekennzeichnet, dass** die eine oder mehrere Feuerungseinrichtung(en) (11) der ersten Produktionsanlage (2) abgasseitig über eine vierte Leitungsverbindung (13) mit der zweiten Abgasreinigungsvorrichtung (14) und/oder mit einer den CO₂-haltigen Abgasstrom (15) des Kraftwerks (9) führenden Leitungsverbindung (16) in medienführender Leitungsverbindung steht/stehen, über welche der zweiten Abgasreinigungsvorrichtung (14) zumindest ein Teil der in der oder den Feuerungseinrichtung(en) (11) und/oder dem Kraftwerk (9) entstehenden CO₂-haltigen Abgasströme (15,17), vorzugsweise die CO₂-haltigen Abgasströme insgesamt, zuführbar ist.

6. Industrielle Produktionsanlage (1) nach einem der vorhergehenden Ansprüche, **dadurch gekennzeichnet, dass** die Gasaufbereitungsanlage (4) als Druckwechseladsorptionsanlage ausgebildet ist.

7. Industrielle Produktionsanlage (1) nach einem der vorhergehenden Ansprüche, **dadurch gekennzeichnet, dass** die einen CO₂-reichen Gasstrom erzeugende Einrichtung (19) als CO₂ mittels eines Absorptionsmittels aus dem CO₂-haltigen Abgasstrom (15, 17) des Kraftwerks (9) und/oder der einen oder mehreren Feuerungseinrichtung(en) (11) waschende Post Combustion Capture (PCC)-Anlage ausgebildet ist.

8. Industrielle Produktionsanlage (1) nach einem der vorhergehenden Ansprüche, **dadurch gekennzeichnet, dass** die aus einem kohlenstoffhaltigen Einsatzstoff ein CO₂-armes und H₂-reiches Produktgas oder Abgas erzeugende erste Produktionsanlage (2) ein Kokereigas erzeugender Koksofen oder eine ein kohlenstoffhaltiges Ausgangsmaterial vergasende oder pyrolysierende oder torrefizierende Anlage ist.

9. Industrielle Produktionsanlage (1) nach einem der vorhergehenden Ansprüche, **dadurch gekennzeichnet, dass** sie ein Hüttenwerk und/oder ein Stahlwerk oder ein Zementwerk, insbesondere einen Drehrohrofen, oder eine oder mehrere Anlagen zur Herstellung von schmelzflüssigem Glas oder ein Chemiewerk oder eine oder mehrere Papierherstellungsanlagen umfasst oder mit diesen/diesem/dieser in einer einen CO₂-haltigen Gasstrom führenden Leitungsverbindung steht.

10. Verfahren zum zumindest im Wesentlichen CO₂-freien Betrieb einer aus einem kohlenstoffhaltigen Einsatzstoff ein CO₂-armes und H₂-reiches Produktgas oder Abgas erzeugenden ersten Produktionsanlage (2) mit einer zugeordneten ersten Abgasreinigungsvorrichtung (3) und einer zugeordneten zweiten Abgasreinigungsvorrichtung (14), insbesondere zum Betrieb einer industriellen Produktionsanlage (1) nach einem der Ansprüche 1 - 9,
**dadurch gekennzeichnet,**
**dass** das CO₂-arme und H₂-reiche Produktgas oder Abgas über eine erste, mit der ersten Abgasreinigungsvorrichtung (3) verbundene Leitungsverbindung (5) einer das Produktgas oder Abgas in einen kohlenstoffhaltigen, zumindest im Wesentlichen H₂-freien (6) und einen zumindest im Wesentlichen kohlenstofffreien, H₂-reichen (7) Teilgasstrom aufteilenden Gasaufbereitungsanlage (4) zugeführt wird und der im Wesentlichen H₂-freie Teilgasstrom (6) über eine zweite Leitungsverbindung (12) einer oder mehreren Feuerungseinrichtung(en) (11) der ersten Produktionsanlage (2) zumindest teilweise zugeleitet wird,
wobei zumindest ein Teil des in der oder den Feuerungseinrichtung(en) (11) entstehenden CO₂-haltigen Abgasstroms (17) nach Durchlaufen der zweiten Abgasreinigungsvorrichtung (14) zumindest teilweise einer Einrichtung (19) zur Erzeugung eines CO₂-reichen Gasstroms zugeführt wird,
wobei in einer Wasserelektrolyseanlage (24) mittels Wasserelektrolyse Wasserstoff (H₂) sowie Sauerstoff (O₂) und in einer zweiten Produktionsanlage (20) Methanol und/oder Methanolfolgeprodukte erzeugt werden und der zweiten Produktionsanlage (20) einerseits über eine CO₂-Leitungsverbindung (21) der in der den CO₂-reichen Gasstrom erzeugenden Einrichtung (19) erzeugte CO₂-reiche Gasstrom zumindest teilweise zugeführt wird und der andererseits über eine H₂-Leitungsverbindung (23) der von der Gasaufbereitungsanlage (4) erzeugte H₂-reiche Teilgaststrom (7) und/oder der in der Wasserelektrolyseanlage (24) erzeugte Wasserstoff (H₂) zumindest jeweils teilweise zugeführt wird.

11. Verfahren nach Anspruch 10, **dadurch gekennzeichnet, dass** der im Wesentlichen H₂-freie Teilgasstrom (6) über eine dritte Leitungsverbindung (8) zumindest teilweise einem Kraftwerk (9) zugeleitet wird, wobei das Kraftwerk (9) einen Wasser/Dampfkreislauf mit mindestens einem dampfbeaufschlagten Turbosatz mit mindestens einem Generator (10) aufweist und mit kohlenstoffhaltigem Brennstoff befeuert wird sowie sein CO₂-haltiger Abgasstrom (15) zumindest teilweise der zugeordneten zweiten Abgasreinigungsvorrichtung (14) zugeführt wird.

12. Verfahren nach Anspruch 11, **dadurch gekennzeichnet, dass** die Gasaufbereitungsanlage (4) und die Wasserelektolyseanlage (24) in elektrischer Leitungsverbindung (25, 26, 27, 28, 29) sowohl zu dem mindestens einen Generator (10) des Kraftwerks (9) als auch zum öffentlichen Stromnetz (30) stehen und sie wahlweise mit im öffentlichen Stromnetz (30) vorhandenem Strom, insbesondere Überschussstrom, und/oder mit von dem mindestens einen Generator (10) erzeugtem elektrischem Strom betrieben werden.

13. Verfahren nach einem der Ansprüche 10 bis 12, **dadurch gekennzeichnet, dass** die ein CO₂-armes und H₂-reiches Produktgas oder Abgas erzeugende erste Produktionsanlage (2) eine oder mehrere Feuerungseinrichtungen (11) aufweist, der/denen einlassseitig von der Gasaufbereitungsanlage (4) und/oder der dritten Leitungsverbindung (8) zumindest ein Teil des in der Gasaufbereitungsanlage (4) erzeugten kohlenstoffhaltigen, zumindest im Wesentlichen H₂-freien Teilgasstroms (6) als Brennstoff zugeführt wird.

14. Verfahren nach einem der Ansprüche 10 - 13, **dadurch gekennzeichnet, dass** die ein CO₂-armes und H₂-reiches Produktgas oder Abgas erzeugende erste Produktionsanlage (2) eine oder mehrere Feuerungseinrichtungen (11) aufweist, die abgasseitig über eine vierte Leitungsverbindung (13) mit der zweiten Abgasreinigungsvorrichtung (14) und/oder mit einer den CO₂-haltigen Abgasstrom (15) des Kraftwerks (9) führenden Leitungsverbindung (16) in medienführender Leitungsverbindung steht/stehen, über welche der zweiten Abgasreinigungsvorrichtung (14) zumindest ein Teil der in der oder den Feuerungseinrichtung(en) (11) und/oder dem Kraftwerk (9) entstehenden CO₂-haltigen Abgasströme (15, 17), vorzugsweise die CO₂-haltigen Abgasströme insgesamt, zugeführt wird.

## Claims

1. An industrial production plant (1) comprising
a first production plant (2) producing a low-CO₂ and H₂-rich product gas or offgas from a carbon-containing feed with an associated first offgas purification apparatus (3) and an associated second offgas purification apparatus (14),
a gas treatment plant (4) which is connected to the first offgas purification apparatus (3) via a first conduit connection (5) and separates the product gas or offgas into a carbon-containing, at least substantially H₂-free gas substream (6) and an at least substantially carbon-free, H₂-rich gas substream (7), where the substantially H₂-free gas substream (6) can be fed at least partly by means of a second conduit connection (12) to one or more firing device(s) (11) present in the first production plant (2),
a device (19) for producing a CO₂-rich gas stream, into which at least part of the CO₂-containing offgas stream (17) formed in the firing device(s) (11) can be at least partly fed after flowing through the second offgas purification apparatus (14),
a water electrolysis plant (24) producing hydrogen (H₂) and oxygen (O₂) and a second production plant (20) which produces methanol and/or downstream products of methanol and is firstly connected via the CO₂ conduit connection (21) to the device (19) producing the CO₂-rich gas stream and to which the CO₂-rich gas stream can be at least partly fed by means of this CO₂ conduit connection (21) and, is secondly connected via the H₂-conduit connection (23) to the gas treatment plant (4) and the water electrolysis plant (24) and into which the H₂-rich gas substream (7) produced in the gas treatment plant (4) and/or the hydrogen (H₂) produced in the water electrolysis plant (24) can respectively be at least partly fed by means of this H₂ conduit connection (23).

2. The industrial production plant (1) as claimed in claim 1, **characterized in that** it comprises a power station (9) which is fired using a carbon-containing fuel and has a water/steam circuit which comprises at least one steam-powered turbo set having at least one generator (10) and is in media-conducting conduit connection with the gas treatment plant (4) via a third conduit connection (8), by means of which the substantially H₂-free gas substream (6) can be at least partly fed to the power station (9).

3. The industrial production plant (1) as claimed in claim 2, **characterized in that** the gas treatment plant (4) and the water electrolysis plant (24) have an electrical connection (25, 26, 27, 28, 29) both to the at least one generator (10) of the power station (9) and to the public power grid (30) and optionally can be operated using power present in the public power grid (30), in particular surplus power, and/or using electric power generated by the at least one generator (10).

4. The industrial production plant (1) as claimed in any of the preceding claims, **characterized in that** the one or more firing device(s) (11) of the first production plant (2) is/are, on the inlet side, in media-conducting conduit connection with the gas treatment plant (4) via the second conduit connection (12) and/or the third conduit connection (8), by means of which at least part of the carbon-containing, at least substantially H₂-free gas substream (6) produced in the gas treatment plant (4) can be fed as fuel to the firing device(s) (11).

5. The industrial production plant (1) as claimed in any of the preceding claims, **characterized in that** the one or more firing device(s) (11) of the first production plant (2) is/are, on the offgas side, in media-conducting conduit connection via a fourth conduit connection (13) with the second offgas purification apparatus (14) and/or with a conduit connection (16) conducting the CO₂-containing offgas stream (15) of the power station (9), by means of which at least part of the CO₂-containing offgas streams (15, 17), preferably all the CO₂-containing gas streams, formed in the firing device(s) (11) and/or the power station (9) can be fed to the second offgas purification apparatus (14).

6. The industrial production plant (1) as claimed in any of the preceding claims, **characterized in that** the gas treatment plant (4) is configured as a pressure swing adsorption plant.

7. The industrial production plant (1) as claimed in any of the preceding claims, **characterized in that** the device (19) producing a CO₂-rich gas stream is configured as a post combustion capture (PCC) plant which scrubs out CO₂ from the CO₂-containing offgas stream (15, 17) of the power station (9) and/or the one or more firing device(s) (11) by means of an absorption medium.

8. The industrial production plant (1) as claimed in any of the preceding claims, **characterized in that** the first production plant (2) producing a low-CO₂ and H₂-rich product gas or offgas from a carbon-containing feed is a coke oven producing a coke oven gas or a plant which gasifies or pyrolyzes or torrefies a carbon-containing starting material.

9. The industrial production plant (1) as claimed in any of the preceding claims, **characterized in that** it comprises a smelting works and/or a steelworks or a cement works, in particular a rotary tube furnace, or one or more plants for producing molten glass or a chemical works or one or more paper production plants or is connected thereto by a conduit connection conveying a CO₂-containing gas stream.

10. A method for the at least substantially CO₂-free operation of a first production plant (2) producing a low-CO₂ and H₂-rich product gas or offgas from a carbon-containing feed with an associated first offgas purification apparatus (3) and an associated second offgas purification apparatus (14), in particular for the operation of an industrial production plant (1) as claimed in any of claims 1 - 9,
**characterized in that**
the low-CO₂ and H₂-rich product gas or offgas is fed via a first conduit connection (5) connected to the first offgas purification apparatus (3) to a gas treatment plant (4) which divides the product gas or offgas into a carbon-containing, at least substantially H₂-free gas substream (6) and an at least substantially carbon-free, H₂-rich gas substream (7) and the substantially H₂-free gas substream (6) is at least partly fed via a second conduit connection (12) to one or more firing device(s) (11) of the first production plant (2),
where at least part of the CO₂-containing offgas stream (17) formed in the firing device(s) (11) is, after passing through the second offgas purification apparatus (14), at least partly fed to a device (19) for producing a CO₂-rich gas stream,
where hydrogen (H₂) and oxygen (O₂) are produced by electrolysis of water in a water electrolysis plant (24) and methanol and/or downstream products of methanol are produced in a second production plant (20) and, firstly, the CO₂-rich gas stream produced in the device (19) producing the CO₂-rich gas stream is at least partly fed to the second production plant (20) and, secondly, the H₂-rich gas substream (7) produced by the gas treatment plant (4) and/or the hydrogen (H₂) produced in the water electrolysis plant (24) is in each case at least partly fed via an H₂-conduit connection (23) to the second production plant (20).

11. The method as claimed in claim 10, **characterized in that** the substantially H₂-free gas substream (6) is fed at least partly via a third conduit connection (8) to a power station (9), where the power station (9) has a water/steam circuit with at least one steam-powered turbo set with at least one generator (10) and is fired using a carbon-containing fuel and the CO₂-containing offgas stream (15) therefrom is at least partly fed to the associated second offgas purification apparatus (14).

12. The method as claimed in claim 11, **characterized in that** the gas treatment plant (4) and the water electrolysis plant (24) have an electrical connection (25, 26, 27, 28, 29) both to the at least one generator (10) of the power station (9) and to the public power grid (30) and are optionally operated by means of power present in the public power grid (30), in particular surplus power, and/or using electric power produced by the at least one generator (10).

13. The method as claimed in any of claims 10 to 12, **characterized in that** the first production plant (2) producing a low-CO₂ and H₂-rich product gas or offgas has one or more firing devices (11) to which, on the inlet side, at least part of the carbon-containing, at least substantially H₂-free gas substream (6) is fed as fuel from the gas treatment plant (4) and/or the third conduit connection (8).

14. The method as claimed in any of claims 10 - 13, **characterized in that** the first production plant (2) producing a low-CO₂ and H₂-rich product gas or offgas has one or more firing devices (11) which is/are, on the offgas side, in media-conducting conduit connection via a fourth conduit connection (13) with the second offgas purification apparatus (14) and/or with a conduit connection (16) conducting the CO₂-containing offgas stream (15) of the power station (9), by means of which at least part of the CO₂-containing offgas streams (15, 17), preferably all the CO₂-containing offgas streams, formed in the firing device(s) (11) and/or the power station (9) is fed to the second offgas purification apparatus (14).

## Revendications

1. Installation de production industrielle (1), comprenant
une première installation de production (2), produisant à partir d'une matière première contenant du carbone un produit gazeux ou un gaz d'échappement pauvre en CO₂ et riche en H₂, comportant un premier dispositif associé (3) de purification des gaz d'échappement et un second dispositif associé (14) de purification des gaz d'échappement,
une installation (4) de traitement des gaz, reliée par une première liaison par conduite (5) au premier dispositif (3) de purification des gaz d'échappement, subdivisant le produit gazeux ou le gaz d'échappement en un courant gazeux partiel (6) contenant du carbone, au moins pour l'essentiel exempt de H₂, et en un courant gazeux partiel (7) au moins pour l'essentiel exempt de carbone, riche en H₂, le courant gazeux partiel (6) pour l'essentiel exempt de H₂ pouvant, à l'aide d'une deuxième conduite de liaison (12), être au moins partiellement envoyé à un ou plusieurs dispositifs de chauffe (11), que présente la première installation de production (2),
un dispositif (19) destiné à la production d'un courant gazeux riche en CO₂, à laquelle peut être envoyée, au moins partiellement, une partie du courant de gaz d'échappement (17) contenant du CO₂, produit dans le ou dans les dispositifs de chauffe (11), après avoir traversé le deuxième dispositif (14) de purification des gaz d'échappement,
une installation (24) d'électrolyse de l'eau, produisant de l'hydrogène (H₂) et de l'oxygène (O₂),
et une deuxième installation de production (20), produisant du méthanol et/ou des produits dérivés du méthanol, qui d'une part est reliée, par une liaison par conduite de CO₂ (21), au dispositif (19) produisant le courant gazeux riche en CO₂, et à laquelle, à l'aide de cette liaison par conduite de CO₂ (21), il est possible d'envoyer au moins partiellement le courant gazeux riche en CO₂, et qui d'autre part est relié par une liaison par conduite de H₂ (23) à l'installation (4) de traitement des gaz et à l'installation (24) d'électrolyse de l'eau, et à laquelle, à l'aide de cette liaison par conduite de H₂ (23), il est possible d'envoyer, chacun au moins partiellement, le courant gazeux partiel (7) riche en H₂ et produit dans l'installation (4) de traitement des gaz et/ou l'hydrogène H₂ produit dans l'installation (24) d'électrolyse de l'eau.

2. Installation de production industrielle (1) selon la revendication 1, **caractérisée en ce qu'**elle comprend une centrale (9), chauffée par un combustible contenant du carbone, centrale qui présente un circuit eau/vapeur, qui comprend au moins un turbogénérateur à admission de vapeur, comportant au moins un générateur (10), et qui par l'intermédiaire d'une troisième liaison par conduite (8) est relié, par une liaison par conduite de fluide, à l'aide de laquelle le courant gazeux partiel (6) pour l'essentiel exempt de H₂ peut au moins partiellement être envoyé à la centrale (9), avec l'installation (4) de traitement des gaz.

3. Installation de production industrielle (1) selon la revendication 2, **caractérisée en ce que** l'installation (4) de traitement des gaz et l'installation (24) d'électrolyse de l'eau présentent une liaison par une ligne électrique (25, 26, 27, 28, 29), tant avec l'au moins un générateur (10) de la centrale (9), qu'avec le réseau public (30), et peut, au choix, être exploitée avec le courant présent dans le réseau public (30), en particulier le surplus de courant, et/ou avec le courant électrique produit par l'au moins un générateur (10).

4. Installation de production industrielle (1) selon l'une des revendications précédentes, **caractérisée en ce que** le ou les dispositifs de chauffe (11) de la première installation de production (2) sont, côté entrée, par l'intermédiaire de la deuxième liaison par conduite (12) et/ou de la troisième liaison par conduite (8), reliés, par liaison par conduite de fluide avec l'installation (4) de traitement des gaz, par l'intermédiaire de laquelle il est possible d'envoyer à la ou aux installations de chauffe (11), en tant que combustible, au moins une partie du courant gazeux partiel (6), produit dans l'installation (4) de traitement des gaz, contenant du carbone, au moins pour l'essentiel exempt de H₂.

5. Installation de production industrielle (1) selon l'une des revendications précédentes, **caractérisée en ce que** le ou les dispositifs de chauffe (11) de la première installation de production (2) sont, côté gaz d'échappement, par l'intermédiaire d'une quatrième liaison par conduite (13), reliés, par liaison par conduite de fluide, au deuxième dispositif (14) de purification des gaz d'échappement et/ou à une liaison par conduite (16) transportant le courant de gaz d'échappement (15), contenant du CO₂, de la centrale (9), liaison par conduite de fluide par l'intermédiaire de laquelle il est possible d'envoyer au deuxième dispositif (14) de purification des gaz d'échappement au moins une partie des courants de gaz d'échappement (15, 17), contenant du CO₂, produits dans le ou les dispositifs de chauffe (11) et/ou dans la centrale (9), de préférence la totalité des courants de gaz d'échappement contenant du CO₂.

6. Installation de production industrielle (1) selon l'une des revendications précédentes, **caractérisée en ce que** l'installation (4) de traitement des gaz est configurée comme une installation d'adsorption modulée en pression.

7. Installation de production industrielle (1) selon l'une des revendications précédentes, **caractérisée en ce que** le dispositif (19) produisant un courant gazeux riche en CO₂ est configuré comme une installation Post Combustion Capture (PCC, captage postcombustion) qui à l'aide d'un agent adsorbant élimine le CO₂ du courant de gaz d'échappement (15, 17) contenant du CO₂ de la centrale (9) et/ou du ou des dispositifs de chauffe (11).

8. Installation de production industrielle (1) selon l'une des revendications précédentes, **caractérisée en ce que** la première installation de production (2) produisant à partir d'une matière première contenant du carbone un produit gazeux ou un gaz d'échappement pauvre en CO₂ et riche en H₂ est un four à coke produisant du gaz de cokerie ou une installation de gazéification ou de pyrolyse ou de torréfaction d'une matière de départ contenant du carbone.

9. Installation de production industrielle (1) selon l'une des revendications précédentes, **caractérisée en ce qu'**elle comprend une usine sidérurgique et/ou une aciérie ou une cimenterie, en particulier un four tubulaire rotatif ou une ou plusieurs installations de fabrication de verre fondu ou une usine chimique ou une ou plusieurs installations de fabrication du papier, ou est reliée, par une liaison par conduite, à ces derniers par une liaison par conduite transportant un courant gazeux contenant du CO₂.

10. Procédé pour l'exploitation, au moins pour l'essentiel exempt de CO₂, d'une première installation de production (2), produisant à partir d'une matière première contenant du carbone un produit gazeux ou un gaz d'échappement pauvre en CO₂ et riche en H₂, comportant un premier dispositif associé (3) de purification des gaz d'échappement et un deuxième dispositif associé (14) de purification des gaz d'échappement, en particulier pour l'exploitation d'une installation de production industrielle (1) selon l'une des revendications 1 à 9,
**caractérisé en ce que**
le produit gazeux ou le gaz d'échappement pauvre en CO₂ et riche en H₂ est, par l'intermédiaire d'une première liaison par conduite (5), relié au premier dispositif (3) de purification des gaz d'échappement, envoyé à une installation (4) de traitement des gaz, qui subdivise le produit gazeux ou le gaz d'échappement en un courant gazeux partiel (6) contenant du carbone, au moins pour l'essentiel exempt de H₂, et en un courant gazeux partiel (7) au moins pour l'essentiel exempt de carbone, riche en H₂, et que le courant gazeux partiel (6) pour l'essentiel exempt de H₂ est, par l'intermédiaire d'une deuxième liaison par conduite (12), au moins partiellement envoyé à un ou plusieurs dispositifs de chauffe (11) de la première installation de production (2),
au moins une partie du courant de gaz d'échappement (17) contenant du CO₂, produite dans le ou les dispositifs de chauffe (11), étant, après avoir traversé le deuxième dispositif de purification des gaz d'échappement (14), au moins partiellement envoyée à un dispositif de production d'un courant gazeux riche en CO₂.
dans une installation (24) d'électrolyse de l'eau, de l'hydrogène (H₂) et de l'oxygène (O₂) étant produits par électrolyse de l'eau, et, dans une deuxième installation de production (20), du méthanol et/ou des produits dérivés du méthanol étant produits, et d'une part, par l'intermédiaire d'une liaison par conduite de CO₂ (21), le courant gazeux riche en CO₂, produit dans le dispositif (19) produisant le courant gazeux riche en CO₂, étant au moins partiellement envoyé à la deuxième installation de production (20), et d'autre part, par l'intermédiaire d'une liaison par conduite de H₂ (23), le courant gazeux partiel (7) riche en H₂, produit par l'installation (4) de traitement des gaz, et/ou l'hydrogène (H₂) produit dans l'installation (24) d'électrolyse de l'eau, étant chacun au moins partiellement envoyé à cette deuxième installation de production.

11. Procédé selon la revendication 10, **caractérisé en ce que** le courant gazeux partiel (6) pour l'essentiel exempt de H₂ est, par l'intermédiaire d'une troisième liaison par conduite (8), envoyé au moins partiellement à une centrale (9), la centrale (9) présentant un circuit eau/vapeur comportant au moins un turbogénérateur à admission de vapeur comportant au moins un générateur (10) et étant chauffée par un combustible contenant du carbone, son courant de gaz d'échappement (15) contenant du CO₂ étant lui aussi au moins partiellement envoyé au deuxième dispositif associé (14) de purification des gaz d'échappement.

12. Procédé selon la revendication 11, **caractérisé en ce que** l'installation (4) de traitement des gaz et l'installation (24) d'électrolyse de l'eau sont reliés par une ligne électrique (25, 26, 27, 28, 29) tant avec l'au moins un générateur (10) de la centrale (9) qu'avec le réseau public (30), et qu'elles sont exploitées au choix avec le courant présent dans le réseau public (30), en particulier le surplus de courant, et/ou avec le courant électrique produit par l'au moins un générateur (10).

13. Procédé selon l'une des revendications 10 à 12, **caractérisé en ce que** la première installation de production (2) produisant un produit gazeux ou un gaz d'échappement pauvre en CO₂ et riche en H₂ présente un ou plusieurs dispositifs de chauffe (11), côté entrée desquels, provenant de l'installation (4) de traitement des gaz et/ou de la troisième liaison par conduite (8), est envoyée en tant que combustible au moins une partie du courant gazeux partiel (6) produit dans l'installation (4) de traitement des gaz, contenant du carbone, au moins pour l'essentiel exempt de H₂.

14. Procédé selon l'une des revendications 10 à 13, **caractérisé en ce que** la première installation de production (2) produisant un produit gazeux ou un gaz d'échappement pauvre en CO₂ et riche en H₂ présente un ou plusieurs dispositifs de chauffe (11), qui côté gaz d'échappement sont reliés par une liaison par conduite de fluide, par l'intermédiaire d'une quatrième liaison par conduite (13), au deuxième dispositif (14) de purification des gaz d'échappement et/ou à une liaison par conduite (16), transportant le courant de gaz d'échappement (15), contenant du CO₂, de la centrale (9), liaison par conduite par l'intermédiaire de laquelle au moins une partie des courants de gaz d'échappement (15, 17) contenant du CO₂, produits dans le ou les dispositifs de chauffe (11) et/ou dans la centrale (9), de préférence la totalité des courants de gaz d'échappement contenant du CO₂, est envoyée au deuxième dispositif (14) de purification des gaz d'échappement.
